Europäisches Patentamt

(19) European Patent Office

Office européen des brevets

(11) EP 0 725 077 A1

(12) EUROPEAN PATENT APPLICATION

(43) Date of publication:
07.08.1996 Bulletin 1996/32

(51) Int. Cl.$^6$: C07K 14/375, A61K 38/16, C12P 21/00

(21) Application number: 96101623.5

(22) Date of filing: 06.02.1996

(84) Designated Contracting States:
DE FR GB

(30) Priority: 06.02.1995 JP 41230/95

(71) Applicant: Kureha Chemical Industry Co., Ltd.
Tokyo 103 (JP)

(72) Inventors:
• Ohara, Minoru
  Tokyo 174 (JP)
• Oguchi, Yoshihara
  Tokyo 176 (JP)
• Matsunaga, Kenichi
  Tokorozawa-shi, Saitama 359 (JP)

(74) Representative: Cohausz & Florack
Patentanwälte
Kanzlerstrasse 8a
40472 Düsseldorf (DE)

(54) Novel glycoproteins, process for preparation thereof, and pharmaceutical composition

(57) A glycoprotein which is prepared by chemically treating an extract from mycelium, broth or fruit body of a fungus belonging to Coriolus, and has following physicochemical properties:
a molecular weight determined by gel chromatography being 5,000 to 1,000,000;
a ratio (proteins/sugars) of an amount of proteins determined by Lowry-Folin method to an amount of sugars determined by phenol-sulfate method being 0.7 to 5.0; and
1→3 glucan accounting for 18 to 100 % in glucans in sugars is disclosed. The glycoprotein is effective to a malignant tumor, or a disease which an immune system or a growth factor is implicated in.

**Description**

BACKGROUND OF THE INVENTION

1. Field of the Invention

The present invention relates to novel glycoproteins, a process for preparation thereof, and pharmaceutical composition, particularly, an anti-tumor agent, immunoregulator, or growth factor inhibitor, containing the glycoprotein as an active ingredient. The glycoproteins of the present invention may be used for treating or preventing a malignant tumor, and various diseases which an immune system or a growth factor is implicated in.

2. Description of the Related Art

It is known that various polysaccharides and protein-bound-polysaccharides obtained from Basidiomycetes exhibit anti-tumor activity (Chihara G: Revista Espanda de Entomologia, 4:85-96, 1984). Some products were found to exhibit a regulatory activity for functions of a biological protective mechanism, mainly an immune system. It is believed that the anti-tumor activity is exhibited through the regulatory activity, i.e., the immunoregulatory function. Therefore, said products, nowadays, are classified under biological response modifiers (BRM).

One of the protein-bound-polysaccharides obtained from the fungus belonging to Coriolus, which is commercially available as "Krestin" (trade mark) from Sankyo Co., Ltd., and generally called "PSK", has been clinically used, and proved to be able to prolong a life of a patient suffering from cancer (Nakazato H, et al.: The Lancet, 343:1122-1126, 1994). Various activities and mechanisms thereof, for example, immunoregulating functions, such as an activity to induce cytokine production (Hirose K, et al.: Lymphokine Res. 9:475-483, 1990), and a function to activate T-cells (Hirai R, et al.: Int. J. Immunopharmac. 15:745-750, 1993) were reported. Recently, it was found that PSK has an activity to inhibit functions of growth factors, such as transforming growth factor $\beta1$ (TGF-$\beta1$) and platelet derived growth factor (PDGF) (Matsunaga K., et al., an article in 53rd General Conference of Japan Cancer Society, p. 455, 1994).

SUMMARY OF THE INVENTION

The inventors of the present invention engaged in intensive studies to various derivatives of the protein-bound-polysaccharides obtained from Basidiomycetes and the biological activities thereof, and as a result, found that the anti-tumor activity, immunoregulatory function and growth-factor-inhibitory function can be remarkably improved by subjecting the protein-bound-polysaccharides to a chemical treatment, particularly, oxidation with periodic acid or a salt thereof. The present invention is based on the findings.

Accordingly, the object of the present invention is to provide a novel glycoprotein having an improved anti-tumor, immunoregulatory and growth-factor-inhibitory activities.

Another object of the present invention is to provide a means effective to treat or prevent a malignant tumor, and various diseases which an immune system or a growth factor is implicated in.

Other objects and advantages will be apparent from the following description.

In accordance with the present invention, there is provided a glycoprotein which is prepared by chemically treating an extract from mycelium, broth or fruit body of a fungus belonging to Coriolus, and has following physicochemical properties:

a molecular weight determined by gel chromatography being 5,000 to 1,000,000;

a ratio (proteins/sugars) of an amount of proteins determined by Lowry-Folin method to an amount of sugars determined by phenol-sulfate method being 0.7 to 5.0; and $1\rightarrow3$ glucan accounting for 18 to 100 % in glucans in sugars.

Further, the present invention relates to a method for preparing said glycoprotein by chemically treating a protein-bound-polysaccharides which is an extract from mycelium, broth or fruit body of a fungus belonging to Coriolus, and which contains $1\rightarrow3$, $1\rightarrow4$ and/or $1\rightarrow6$ glucans and about 5 to 60% by weight of proteins.

Still further, the present invention relates to a pharmaceutical composition, particularly, an anti-tumor, immunoregulatory or growth-factor-inhibitory agent, comprising said glycoprotein as an active ingredient and a pharmaceutically acceptable carrier or dilute.

BRIEF DESCRIPTION OF DRAWINGS

Figure 1 is a graph illustrating an anti-tumor activity of the glycoprotein S1 according to the present invention, in comparison with that of PSK, determined in Example 5.

Figure 2 is a graph illustrating a T-cell-growth-accelerating activity of the glycoprotein S1 according to the present invention, in comparison with that of PSK, determined in Example 6.

Figure 3 is a graph illustrating an IL-1β-production-inducing activity of the glycoprotein S1 according to the present invention, in comparison with that of PSK, determined in Example 7.

Figure 4 is a graph illustrating an IL-6-production-inducing activity of the glycoprotein S1 according to the present invention, in comparison with that of PSK, determined in Example 7.

Figure 5 is a graph illustrating a binding activity of the glycoprotein S1 according to the present invention to TGF-β1, in comparison with that of PSK to TGF-β1, determined in Example 8.

Figure 6 is a graph illustrating a binding activity of the glycoprotein S1 according to the present invention to PDGF, in comparison with that of PSK to PDGF, determined in Example 8.

DESCRIPTION OF THE PREFERRED EMBODIMENTS

The present invention will be explained in detail hereinafter.

The protein-bound-polysaccharide used as a starting material in the preparation of the glycoprotein of the present invention is disclosed in, for example, Japanese Examined Patent Publications (Kokai) No. 46-17149, No. 51-36322, No. 56-14274, No. 56-14275, and No. 56-14276. The protein-bound-polysaccharide is an extract from mycelia, broth, or fruit bodies that is obtained by culturing the fungus belonging to Coriolus, which belongs to Basidiomycetes. The protein-bound-polysaccharide contains about 18 to 38 % by weight of proteins, and has a molecular weight (ultracentrifugal method) of not less than 5,000, preferably 5,000 to 1,000,000.

A typical example of the protein-bound-polysaccharide is called PSK, which is commercially available as "Krestin" (trade mark) from Sankyo Co., Ltd. Further, the protein-bound-polysaccharide is described in "Saikin no Shinyaku (Recent New Medicines)", Vol. 28, 14-16, 1977; and Vol. 29, 96-101, 1978; and "Iyakuhin Yoran (Handbook of Medicines)", 6th Ed., 1346, 1979, published by Yakuji Jiho Publishing Co.

The properties of PSK are as follows:

PSK may be prepared by extracting the mycelia of Coriolus versicolor (Fr.) Quél CM101 strain [FERM-P2412 (ATCC20547)] with an aqueous solvent, such as hot water or alkaline solution (e.g., a solution of hydroxide of alkali metal, such as sodium hydroxide), purifying and then drying the extract. The sugar portion in the major fractions is β-D-glucan. The glucan has a branched structure containing 1→3, 1→4 and 1→6 bonds, and mainly comprises glucose and mannose. Further, PSK contains about 18 to 38 % by weight of proteins. In the amino acid composition of the proteins, there are many acidic amino acids, such as aspartic and glutamic acids, and many neutral amino acids, such as valine and leucine, but a few basic amino acids, such as lysine and arginine. PSK is soluble in water, but hardly soluble in methanol, pyridine, chloroform, benzene or hexane. PSK begins to be gradually decomposed at about 120 °C.

As a starting material of the glycoprotein according to the present invention, the extract from the fungi strains (FERM-P Nos. 2413 to 2426) belonging to Coriolus other than Coriolus versicolor (Fr.) Quél CM101 strain as above, Coriolus consors (Berk.) Imaz., Coriolus pubescens (Fr.) Quél., Coriolus biformis (Kiotz.) Pat., Coriolus hirsutus (Fr.) Quél., Coriolus conchifer (Schw.) Pat., Coriolus pargamenus (Fr.) Pat. and the like may be used.

The desired physiologically active substance, glycoprotein, may be prepared by chemically treating the above extract as follows. The chemical treatment comprises steps of, for example, oxidation, reduction, mild hydrolysis, and removing of the low-molecular products.

The oxidation of the extract causes cleavages of glycol moieties, and may be carried out, using a periodic acid or a salt thereof. More specifically, the extract is dissolved in an aqueous solution or a buffer (neutral pH) containing 0.01 M to 0.2 M sodium metaperiodate, and treated at 0 to 40 °C for 24 hours to 60 days to oxidize sugar portions. Then, the product is treated with a reducing agent, such as sodium borohydride. The resulting protein-bound-polysaccharide polyalcohol is hydrolyzed mildly at 4 to 80 °C for 1 to 96 hours with 0.01 N to 1 N inorganic acid (such as sulfuric or hydrochloric acid) or organic acid (such as oxalic or formic acid), and then, oxidized sugar-chain portions are removed. The desired biologically active substance, glycoprotein may be obtained by carrying out the sequential steps from the oxidation with sodium metaperiodate to the moderate hydrolysis once or repeating the sequential steps several times to decrease 1→4 bound and 1→6 bound sugar portions, and then, removing the low-molecular products.

The biologically active substance, glycoprotein, according to the present invention has the following physicochemical properties:

a molecular weight determined by gel chromatography, for example, gel chromatography of dextran gel or agalose gel (Superose 6: Pharmacia), is 5,000 to 1,000,000;

a ratio (proteins/sugars) of an amount of proteins determined by the Lowry-Folin method [O. H. Lowry, N. J. Rosebrough, A. L. Farr, and R. J. Randall, J. Randall, J. Biol. Chem., 193, 265 (1951)] to an amount of sugars determined by the phenol-sulfate method (J. E. Hodge, B. T. Hofreiter, "Methods in carbohydrate chemistry", Vol.1, Ed. by R. L. Whistler, M. I. Wolfrom, Academic Press, Inc., New York, N. Y., 1962, p.388) is 0.7 to 5.0; and

1→3 glucan, particularly β1→3 glucan, accounts for 18 to 100 % by weight in glucans in sugars.

When the glycoprotein of the present invention is hydrolyzed with hydrochloric acid to analyze a composition of the amino acids, aspartic acid, threonine, serine, glutamic acid, proline, glycine, alanine, cysteine, valine, methionine, isoleucine, leucine, tyrosine, phenylalanine, lysine, histidine and arginine are detected. Of the amino acids, aspartic acid, threonine, serine, glutamic acid, glycine, alanine, phenylalanine, valine, isoleucine and leucine account for 75 mole % in the total amino acids detected. When the glycoprotein is hydrolyzed with sulfuric acid to analyze a composition of the sugars, fucose, xylose, mannose, galactose and glucose are detected. Of the sugars, glucose, mannose and xylose account for 90 % by weight or more in the total sugars.

An anti-tumor activity, interleukin-1$\beta$ (IL-1$\beta$) and interleukin-6 (IL-6) inducing activity, T-cell-growth inducing activity, and TGF-$\beta$1 and PDGF inhibitory activity of the the glycoprotein of the present invention can become two to 10 times or more as high as those of the protein-bound-polysaccharide which is obtained from Basidiomycetes, but is not chemically treated as above. More particularly, the biologically active substance having higher activities can be obtained by treating the extract obtained from Basidiomycetes with periodic acid to selectively decrease 1$\rightarrow$4 bound sugar portions, particularly $\beta$1$\rightarrow$4 bound sugar portions, and 1$\rightarrow$6 bound sugar portions, particularly $\beta$1$\rightarrow$6 bound sugar portions.

It is suggested that PSK is useful to various diseases. The biologically active substance of the present invention exhibits activities as high as those of PSK, at a dose smaller than that of PSK. Therefore, the biologically active substance, the glycoprotein, of the present invention would be applied to various diseases to which PSK is useful, for example, a malignant tumor, infectious diseases by bacterium, fungus, virus, protozoan, or the like, autoimmune diseases, immunosuppressive symptoms, fibroid lung, hepatic fibrosis, glomerulonephritis, scleroderma, arteriosclerosis, or the like.

When the glycoprotein of the present invention is administered to an animal, particularly a mammal including human, as an active ingredient in a pharmaceutical composition, particularly, an anti-tumor agent, immunoregulatory agent (such as an IL-1$\beta$ or IL-6 inducing agent, or a T-cell growth inducing agent), or a growth-factor inhibitor (such as a TGF-$\beta$1 or PDGF inhibitor), the glycoprotein may be formulated into a composition for various routes, such as a composition for oral administration including sublingual administration, injections for subcutaneous, intravenous, or intramuscular administration, or a suppository for rectal administration. The composition for oral administration is, for example, tablet, granule, powder, or capsule. The composition may contain a binder, excipient, lubricant, disintegrating agent, or wetting agent. The oral liquid composition may be in the form of mixture for internal use, shaking mixture, suspension, emulsion, or syrup, or in the form of a dry product which should be re-dissolved when used. Further, the above liquid composition may contain an additive or preservating agent. A parenteral composition, such as injection, suppository, or ointment, may contain an additive, such as a stabilizing agent, buffer, preservating agent, or isotonicity. The composition may be in the form of an aqueous solution, suspension, solution, or emulsion in oily or aqueous vehicle. Alternatively, the active ingredient may be in the form of powder which should be re-dissolved in a suitable vehicle, such as sterilized water without a pyrogenic substance, when used.

The dose may vary with administration routes, ages, individual differences, or symptoms, but in general, 0.05 to 500 mg/kg weight/day. In an oral administration, a dose of 2 to 500 mg may be administered once or divided into two or three.

Protein-bound-polysaccharides, particularly PSK, obtained from Basidiomycetes contain sugar portions and protein portions. The sugar portions are mainly composed of glucose. It is believed that sugars are bound to proteins by O-glycoside and N-glycoside bonds. NMR analysis of the glucan portions revealed that PSK is a polymer containing various bonds, such as $\beta$1$\rightarrow$3, $\beta$1$\rightarrow$4, and $\beta$1$\rightarrow$6 bonds and so on. As above, it is believed that PSK is a complex containing various structures in a molecule.

The structure or structures which might be implicated in the activities including anti-tumor activity of PSK is not sufficiently elucidated. It is assumed that 1$\rightarrow$4 bound, particularly $\beta$1$\rightarrow$4 bound, and 1$\rightarrow$6 bound, particularly $\beta$1$\rightarrow$6 bound protein portions are decreased by the oxidation of the protein-bound-polysaccharides with periodic acid or a salt thereof, and thus, the biologically activities are remarkably improved. Therefore, whereas PSK is clinically administered at a general oral dose of 3 g/day once to three times a day, the dose of the glycoprotein of the present invention can be administered at a smaller dose.

Examples

The present invention will now be further illustrated by, but is by no means limited to, the following Examples. PIn the following Examples, percentages are by weight, unless stated otherwise.

Example 1: Preparation of Glycoprotein S1 of This Invention

PSK (5 g) was dissolved in 2 liter of an aqueous solution of 0.04 M sodium metaperiodate ($NaIO_4$ : 213.9), and the sugar portions were oxidized at 4 °C in the dark for 14 days while sometimes stirred. Then, ethylene glycol (20 g) was added, and the whole was stirred for several hours to decompose excess sodium metaperiodate and cease the reaction. The resulting solution was neutralized by adding dilute acetic acid. Thereafter, sodium borohydride (5 g) was added and a reduction was carried out overnight to convert aldehyde derivative of the protein-bound-polysaccharide to the corresponding stable alcohol derivative. The resulting derivative was dialyzed on a Visking dialysis tube in running water to remove low-molecular products. Then, sulfuric acid was added to the inner part of dialyzate so that the concentration of sulfuric acid became 0.1 N, and moderate hydrolysis was carried out at room temperature for 24 hours to remove cleaved sugar chains. The solution was neutralized with an aqueous sodium hydroxide solution, concentrated, gel-filtrated through Sephadex G25 column to remove low-molecular products, and lyophilized to obtain 1.7 g of the desired glycoprotein (hereinafter referred to as "S1").

Example 2: Preparation of Glycoprotein S2 of This Invention

The medium [5 % glucose, 0.2 % peptone, 0.3 % yeast extract, 0.1 % $KH_2PO_4$ and 0.1% $MgSO_4$ $7H_2O$] was inoculated with Coriolus versicolor (Fr.) Quél CM101 strain [FERM-P2412 (ATCC20547)]. The inoculated medium was cultured for 10 days, and then, mycelia grown on the medium surface was homogenized together with physiological saline for inoculation. The resulting seed was used to inoculate 1-liter cultivation flasks (100 flasks) each containing 200 ml of the medium having the composition same as above. The inoculated medium was cultured at 25 to 27 °C for 25 days, and the culture was dried to obtain dried mycelium. The yield of the dried mycelium was 2,0 to 4.5 g per 1 cultivation flask.

An aqueous solution (3 liter) of 0.1 N sodium hydroxide was added to the mycelium (100 g), and extraction was performed at 97 °C for 1 hour while stirring. After the extraction was finished, the extract was cooled to room temperature, and filtered through a filter paper. The resulting extract was neutralized with dilute hydrochloric acid, and concentrated by an evaporator. Ammonium sulfate was added to the concentrated liquor (1 liter) so that the ammonium sulfate became saturated, and the precipitate obtained by salting-out was collected by a centrifuge. The precipitate was re-dissolved in distilled water, and the solution was treated by dialysis and ultrafiltration to remove low-molecular products having molecular weights of 5,000 or less. Then, the product was lyophilized to obtain 15 g of the powdered protein-bound-polysaccharide (hereinafter referred to as "PP").

The protein-bound-polysaccharide PP (5 g) was dissolved in an aqueous solution (2 liter) of 0.08 M sodium metaperiodate ($NaIO_4$), and the sugar portions were oxidized at 22 °C in the dark for 7 days while sometimes stirred. Then, the procedures of reduction, moderate hydrolysis, removing of salts, and lyophilization as in Example 1 were repeated to obtain 1.4 g of the desired product (hereinafter referred to as "S2").

Example 3: Preparation of Glycoprotein S3 of This Invention

The protein-bound-polysaccharide PP (5 g) prepared in Example 2 was dissolved in an aqueous solution (2 liter) of 0.04 M sodium metaperiodate ($NaIO_4$), and the sugar portions were oxidized at 25 °C in the dark for 14 days while sometimes stirred. Then, the procedures of reduction, moderate hydrolysis, removing of salts, and lyophilization as in Example 1 were repeated to obtain 1.6 g of the desired product (hereinafter referred to as "S3").

Example 4: Physicochemical Analyses of Glycoproteins of This Invention

(1) Elementary analysis

Yanaco CHN Coder Type MT-3 was used in the elementary analysis.

(2) Ratio of proteins to sugars, i.e., proteins/sugars

After the glycoprotein of the present invention was dissolved in distilled water, the contents of the proteins were determined by the Lowry-Folin method (albumin was used as a standard substance), and the contents of the sugars were determined by the phenol-sulfuric acid method (glucose was used as a standard substance).

(3) Composition of sugars

The glycoprotein of the present invention was hydrolyzed in 1 N sulfuric acid at 100 °C for 16 hours. The product was treated by cation exchange (AS6 column) chromatography using Bio-LC (Dionex). A pulsed amperometric detector was used to detect, identify and quantitatively determine sugars. The results are listed by percentage by weight.

(4) Composition of amino acids

The glycoprotein of the present invention was hydrolyzed in 6 N hydrochloric acid at 110 °C for 22 hours. The product was determined by a ninhydrin colorimetry using an amino acid analyzer (Hitachi L-8500). The results are calculated as mol %.

(5) Quantitative analysis of β1,3 glucan

The glycoprotein of the present invention was dissolved in $D_2O$ to perform deuteration. Sodium-3-trimethylsilylpropionate-2,2,3,3-D4 (TSP) was used as an internal standard, and NMR was measured at 90 °C at 500Hz, using an NMR analyzer, JNM-GSX500 (Nihon Denshi) to determine signals caused by protons ($\alpha$1, 4 bond: 5.35 ± 0.01ppm, $\alpha$1, 3 bond: 5.27 ± 0.01ppm, $\alpha$1, 6 bond: 5.00 ± 0.01, β1, 3 bond: 4.77 ± 0.01, β1, 4 bond: 4.55 ± 0.01ppm, β1, 6 bond: 4.52 ± 0.01ppm). The ratio of β1, 3 glucan in 1, 3 glucan is shown in percentage to the above bonds.

(6) Determination of molecular weight

The glycoprotein of the present invention was dissolved in distilled water, and the solution was treated by gel chromatography using Superose 6 (Pharmacia). Then, the molecular weight was calculated from the eluting distance.

The results for the glycoproteins S1, S2 and S3 are shown in the following Table 1.

Table 1

|  |  | Found Values | | |
|---|---|---|---|---|
| Analyses | | S1 | S2 | S3 |
| (1) Elementary analysis | | | | |
|  | C% | 44.4 | 43.7 | 46.8 |
|  | H% | 5.9 | 6.2 | 6.0 |
|  | N% | 6.7 | 5.0 | 8.9 |
| (2) Proteins/sugars ratio | | | | |
|  |  | 1.7 | 1.3 | 3.3 |
| (3) Sugar composition (%) | | | | |
| (a) glucose | | 78 | 69 | 80 |
| (b) mannose | | 18 | 21 | 14 |
| (c) galactose | | 0 | 2 | 1 |
| (d) xylose | | 3 | 4 | 3 |
| (e) fucose | | 1 | 4 | 2 |
| a+b+d/Total of a to e | | 99 | 94 | 97 |
| (4) Amino acid composition (%) | | | | |
| (a) aspartic acid | | 12.2 | 10.4 | 16.2 |
| (b) threonine | | 4.6 | 3.8 | 1.8 |
| (c) serine | | 4.6 | 3.0 | 2.7 |
| (d) glutamic acid | | 13.1 | 10.7 | 10.7 |
| (e) glycine | | 11.5 | 11.6 | 24.3 |
| (f) alanine | | 3.1 | 12.2 | 15.0 |
| (g) valine | | 8.6 | 8.7 | 3.2 |
| (h) 1/2 cystine | | 0.0 | 0.2 | 1.3 |
| (i) methionine | | 0.2 | 0.8 | 0.6 |
| (j) isoleucine | | 5.9 | 6.6 | 2.1 |
| (k) leucine | | 9.9 | 12.1 | 3.9 |
| (l) tyrosine | | 0.6 | 1.7 | 1.7 |
| (m) phenylalanine | | 4.2 | 5.7 | 4.6 |
| (n) lysine | | 3.3 | 2.5 | 0.7 |
| (o) histidine | | 0.9 | 1.3 | 0.9 |
| (p) arginine | | 1.7 | 2.1 | 0.6 |
| (q) proline | | 5.8 | 6.7 | 8.8 |
| a+b+c+d+e+f+g+j+k+m/Total of a to q | | | | |
|  |  | 87.7 | 84.8 | 84.5 |
| (5) Amount of $\beta$1,3 glucan (Total glucan;%) | | | | |
|  |  | 65 | 100 | 26 |
| (6) Molecular weight | | | | |
|  |  | 5,000-1,000,000 | 5,000-1,000,000 | 5,000-1,000,000 |

Example 5: Anti-tumor activity

Cells ($1\times10^6$) of Sarcoma-180 [obtained from The Kitasato Institute; tumor cell line intraperitoneally subcultured in Jcl:ICR mouce (CLEA Japan, Inc.)] were subcutaneously transplanted into 5-week-old female Jcl:ICR mice (one group consisting of 10 mice) to produce tumor-carrying mice. A predetermined amount of each of the glycoprotein S1 of the present invention and the protein-bound-polysaccharide (PSK) was dissolved in physiological saline, and intraperitoneally administered at the dose of 0.1 ml/10 g of weight of mouse, from the next day of the transplantation, three times a week, and 10 times in total. To tumor-carrying control mice, physiological saline was intraperitoneally administered at the dose of 0.1 ml/10 g of weight of mouse. On the 25th day after the transplantation, mice were sacrificed, tumors were taken, and the weights were measured by a weighing machine. The anti-tumor activities of the active substances were calculated by the following equation (1):

$$\text{Anti-tumor activity (\%)} = [(CW - TW)/CW] \times 100 \tag{1}$$

wherein CW is an average weight (g) of the tumor in the control mouse, and TW is an average weight (g) of the tumor in the mouse to which the active substance is administered.

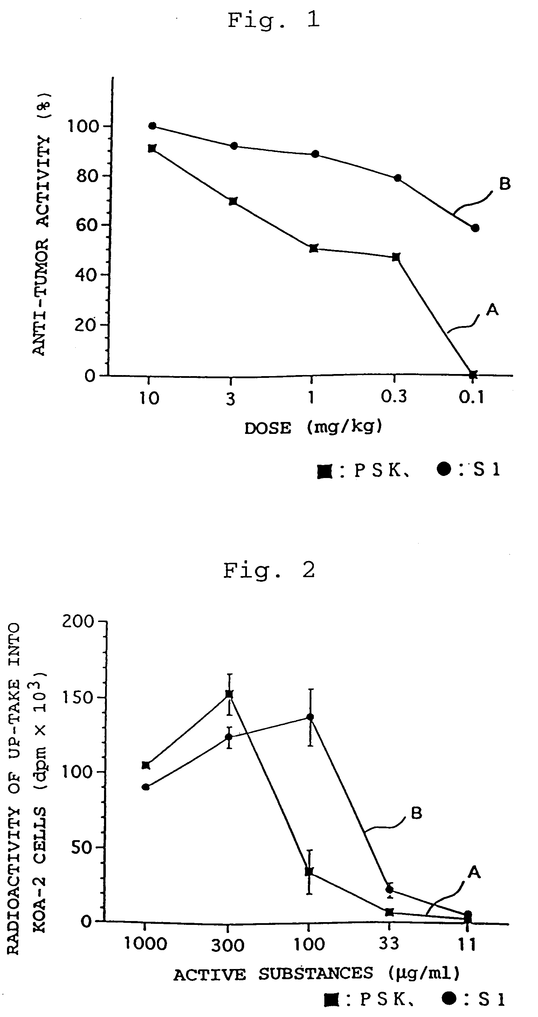

As shown in Fig. 1, the anti-tumor activity (B in Fig. 1) of the glycoprotein S1 of the present invention is apparently superior over the activity (A in Fig. 1) of the protein-bound-polysaccharide (PSK). Further, Table 2 shows the anti-tumor activities of the glycoproteins S2 and S3 of the present invention, which are superior over the activity of the starting material PP.

Table 2

|  | Anti-tumor activity (dosage: 0.3 mg/kg) |
|---|---|
| PP | 28% |
| S2 | 78% |
| S3 | 80% |

Example 6: Immunoregulating activity (activity for promoting T-cell proliferation)

In the present Example, the function of the glycoproteins of the present invention to T-cell clone, KOA-2 (FERM-BP4985), which was isolated from a lymphonodus of BALB/c mouse immunized with PSK, can be cultured in vitro, and can be grown in response to PSK. KOA-2 ($2 \times 10^4$ cells), mitomycin-C-treated BALB/c mouse splenic cells ($5 \times 10^5$ cells) [cells whose proliferating ability has been lost by treating a splenic cell with 50 µg/ml of mitomycin-C at 37 °C for 30 minutes], and the glycoprotein S1 of the present invention or PSK solution were added to wells in 96 well culture microplate (Falcon 3072, Japan Becton Dickinson Co., Ltd.) so that the amount of the total culture (RPMI 1640 containing 10 % bovine fetal serum) became 200 µl/well, and the cultivation was carried out in 5 % $CO_2$ incubator at 37 °C for 3 days. At the time of 6 hours before the end of the cultivation, 18.5 KBq/well of $^3$H-labeled thymidine (Amersham) was added.

The reason why the mitomycin-C-treated BALB/c mouse splenic cells were used is as follows:

Cells having an antigen-presenting ability, such as T-cell or macrophage, take up PSK or the glycoprotein S1 of the present invention, and present the antigen portion of PSK or the glycoprotein S1 on the surface thereof in the form binding to a class II histocompatibility antigen. KOA-2 reacts with the antigen portion of PSK or the glycoprotein S1 bound to the histocompatibility antigen, and proliferates. Therefore, the proliferation of KOA-2 requires the antigen-presenting cell. Further, the splenic cells used had been treated with mitomycin-C, because PSK and the glycoprotein S1 of the present invention have the function to promote also the proliferation of splenic cells, and thus, it was necessary to avoid the proliferation reaction of the splenic cells in the experiment of determining proliferation of KOA-2.

After the cultivation was finished, cells in the wells were collected on a filter by a cell harvester, and washed. The filter was dried, and placed into a vial. After a liquid scintillator was added, a radioactivity taken-up by the cells was measured by a liquid scintillation counter. The results are shown in Fig. 2. It is apparent therefrom that the optimum concentration of PSK added (A in Fig. 2) was 300 µg/ml, whereas that of the glycoprotein S1 of the present invention was 100 µg/ml, and thus, the concentration of the glycoprotein S1 is one-third as large as the concentration of PSK at which the comparable activities can be exhibited.

Further, as shown in Table 3, the activities of the glycoproteins S2 and S3 were higher than that of PP.

Table 3

|  | Growth of KOA-2 cells (100 μg/ml) (Up-take of $^3$H-labeled thymidine; dpm) |
| --- | --- |
| PP | 41,500 |
| S2 | 160,800 |
| S3 | 155,000 |

KOA-2 used in this Example was deposited in the international depositary authority under the Budapest Treaty, the National Institute of Bioscience and Human-Technology Agency of Industrial Science and Technology (address: 1-3, Higashi 1-chome, Tsukuba-shi, Ibaraki, 305, Japan), on January 27, 1995.

Example 7: Immunoregulating activity (Effect for inducing immnoregulatory cytokines production)

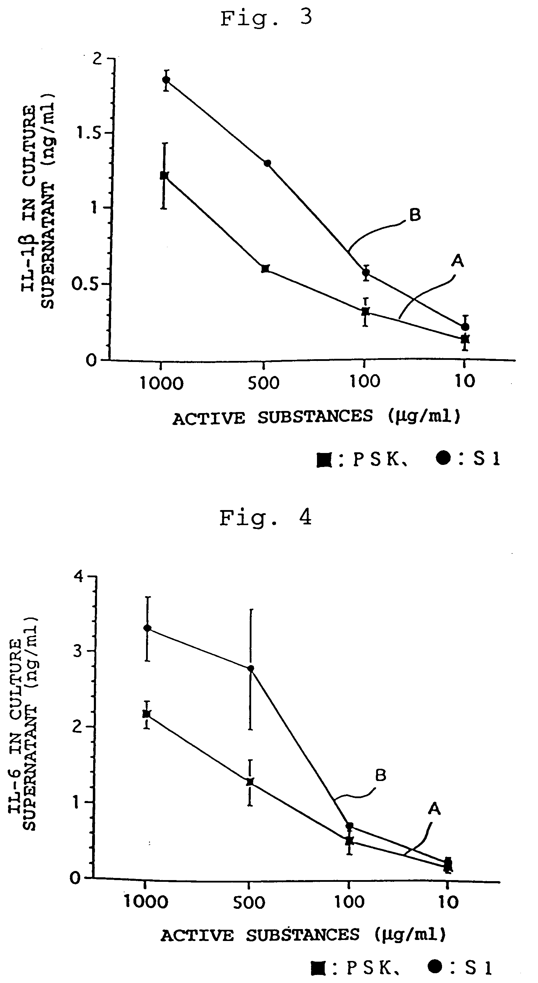

To 30 ml of peripheral blood (with heparin) taken from a healthy adult (48 years old, man), an equal amount of Hanks' solution was added. After thoroughly stirred, the mixture was stratified on the Ficoll-Paque solution (Farmacia) which had been placed in the test tube. The tube was centrifuged at 2000 rpm for 15 minutes, and the monocyte fraction in the middle layer were collected. The cells of the monocyte fraction were washed with Hanks' solution three times, and suspended in the RPMI 1640 medium containing 10 % bovine fetal serum in the concentration of $1 \times 10^6$ celles/ml. The cells (0.1 ml each) were added to the wells in 96 well culture microplate (Falcon 3072, Japan Becton Dickinson Co., Ltd.), and then, the glycoprotein S1 of the present invention or PSK solution were added thereto so that the amount of the total culture (RPMI 1640 containing 10 % bovine fetal serum) became 200 μl/well. The cultivation was carried out in 5 % $CO_2$ incubator at 37 °C for 24 hours. Thereafter, the culture supernatant was separated by centrifuge, and the amount of cytokines was measured using a commercially available enzyme-immunoassay kits (the kit by Otsuka Seiyaku for interleukin 1β, and the kit by Research and Diagnostic System for interleukin 6). The results are shown in Figs. 3 and 4. It is apparent therefrom that the interleukin 1β production (B in Fig. 3) and interleukin 6 production (B in Fig. 4) when the glycoprotein S1 was added were significantly high, in comparison with the interleukin 1β production (A in Fig. 3) and interleukin 6 production (A in Fig. 4) when PSK was added.

Further, the activities of the glycoproteins S2 and S3 were higher than that of PP, as shown in Table 4.

Table 4

|  | Contents (ng/ml) in supernatant of culture (500μl/ml) | |
| --- | --- | --- |
|  | interleukin 1β | interleukin 6 |
| PP | 0.6 | 1.3 |
| S2 | 1.5 | 2.8 |
| S3 | 1.4 | 2.6 |

Example 8: Inhibitory activity against growth factors

To a phosphate buffer (pH 7.4) containing 0.2 % bovine serum albumin, human recombinant TGF-β1 (Research and Diagnostic System) or PDGF (Research and Diagnostic System) were added so that the amount of the solution became 100 ng/ml. To a test tube, 0.2 ml of the resulting solution, and then, 0.2 ml of a solution of the predetermined amount of the glycoprotein S1 or PSK dissolved in a phosphate buffer containing 0.2 % bovine serum albumin were added. Then, the reaction was performed at 22 °C for 3 hours. After the reaction was completed, TGF-β1 or PDGF in the reaction mixture was determined using a commercially available enzyme-immunoassay kits (the kit by Amersham for TGF-β1, and the kit by Research and Diagnostic System for PDGF). The TGF-β1- or PDGF-binding activities were calculated by the following equation (2):

$$\text{Binding activity (\%)} = [(CV - TV)/CV] \times 100 \qquad (2)$$

wherein CV is a value found for the control group, and TV is a value found for a group wherein the active substances.

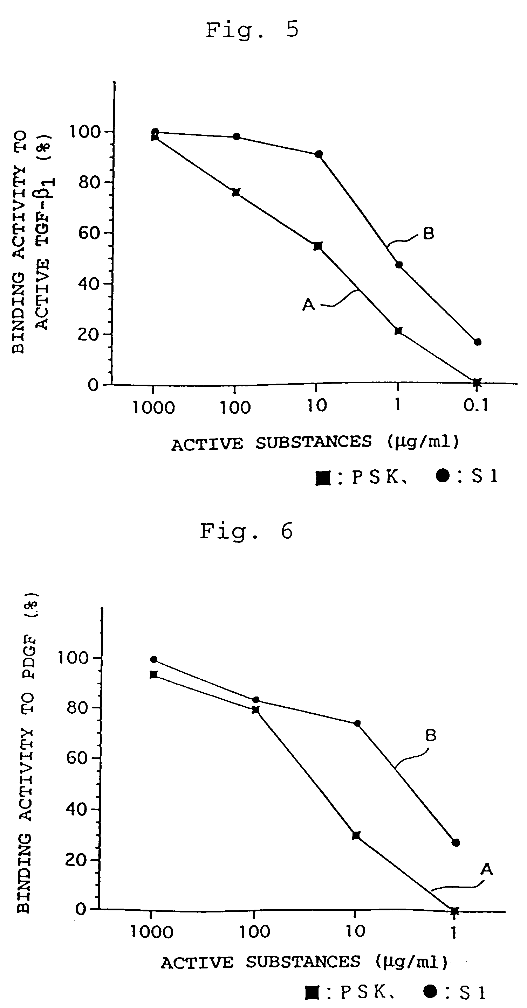

As shown in Fig. 5 (TGF-β1) and Fig. 6 (PDGF), the direct binding activities to TGF-β1 (B in Fig. 5) and PDGF (B in Fig. 6) of the glycoprotein S1 are apparently superior over the corresponding activities (A in Figs. 5 and 6) of PSK.

Further, the activities of the glycoproteins S2 and S3 are superior over the activity of the starting material PP, as shown in Table 5.

Table 5

| | Binding activity (%) (10 μg/ml of substances added) | |
|---|---|---|
| | TGF-β1 | PDGF |
| PP | 25 | 21 |
| S2 | 90 | 76 |
| S3 | 88 | 70 |

As above, in accordance with the glycoprotein of the present invention, physiological activities are remarkably improved by the oxidation of the protein-bound-polysaccharides from the fungus belonging to Coriolus, with periodic acid to decrease the 1→4 and 1→6 bound sugar portions. Therefore, the the glycoprotein of the present invention can be used as anti-malignant tumor agent, immnoregulating agent (such as an inducing agent for IL-1β, IL-6 or T-cell growth), or growth-factor inhibitory agent (such as an inhibiting agent for TGF-β1 or PDGF), while decreasing the dose in comparison with that of the protein-bound-polysaccharides.

Although the present invention has been described with reference to specific embodiments, various changes and modifications obvious to those skilled in the art are deemed to be within the sprit, scope, and concept of the invention.

## Claims

1. A glycoprotein which is prepared by chemically treating an extract from mycelium, broth or fruit body of a fungus belonging to Coriolus, and has following physicochemical properties:
   a molecular weight determined by gel chromatography being 5,000 to 1,000,000;
   a ratio (proteins/sugars) of an amount of proteins determined by Lowry-Folin method to an amount of sugars determined by phenol-sulfate method being 0.7 to 5.0; and
   1→3 glucan accounting for 18 to 100 % in glucans in sugars.

2. The glycoprotein according to claim 1, wherein, by an analysis of amino acids in hydrolyzates with hydrochloric acid, aspartic acid, threonine, serine, glutamic acid, proline, glycine, alanine, cysteine, valine, methionine, isoleucine, leucine, tyrosine, phenylalanine, lysine, histidine and arginine are detected; and aspartic acid, threonine, serine, glutamic acid, glycine, alanine, phenylalanine, valine, isoleucine and leucine account for 75 mole % or more in the total amino acids detected.

3. The glycoprotein according to claim 1, wherein, by an analysis of sugars in hydrolyzates with sulfuric acid, fucose, xylose, mannose, galactose and glucose are detected; and glucose, mannose and xylose account for 90 % by weight or more in the total sugars detected.

4. The glycoprotein according to claim 1, wherein said extract from mycelium, broth or fruit body of a fungus belonging to Coriolus is obtained by extraction with hot water or an alkaline solution.

5. The glycoprotein according to claim 1, wherein said extract from mycelium, broth or fruit body of a fungus belonging to Coriolus is obtained by extraction with hot water or an alkaline solution, and a protein-bound-polysaccharide containing 1→3, 1→4 and 1→6 bound glucans and about 5 to 60 % by weight of proteins.

6. The glycoprotein according to claim 1, wherein said chemical treatment comprises oxidation with a periodic acid or a salt thereof.

7. The glycoprotein according to claim 1, wherein said extract from mycelium, broth or fruit body of a fungus belonging to Coriolus is PSK.

8. A process for preparing a glycoprotein, comprising chemically treating an extract from mycelium, broth or fruit body of a fungus belonging to Coriolus, said extract being a protein-bound-polysaccharide containing $1\rightarrow3$, $1\rightarrow4$ and $1\rightarrow6$ bound glucans and about 5 to 60 % by weight of proteins.

9. A pharmaceutical composition comprising a glycoprotein which is prepared by chemically treating an extract from mycelium, broth or fruit body of a fungus belonging to Coriolus, and has following physicochemical properties:
a molecular weight determined by gel chromatography being 5,000 to 1,000,000;
a ratio (proteins/sugars) of an amount of proteins determined by Lowry-Folin method to an amount of sugars determined by phenol-sulfate method being 0.7 to 5.0; and
$1\rightarrow3$ glucan accounting for 18 to 100 % in glucans in sugars;
and a pharmaceutically acceptable carrier.

10. The pharmaceutical composition according to claim 9, which is an anti-tumor agent.

11. The pharmaceutical composition according to claim 9, which is an immunoregulating agent.

12. The pharmaceutical composition according to claim 9, which is an agent for inhibiting a growth-factor.

13. Use of a glycoprotein which is prepared by chemically treating an extract from mycelium, broth or fruit body of a fungus belonging to Coriolus, and has following physicochemical properties:
a molecular weight determined by gel chromatography being 5,000 to 1,000,000;
a ratio (proteins/sugars) of an amount of proteins determined by Lowry-Folin method to an amount of sugars determined by phenol-sulfate method being 0.7 to 5.0; and
$1\rightarrow3$ glucan accounting for 18 to 100 % in glucans in sugars, for preparing a pharmaceutical composition.

Fig. 1

■ : P S K、　● : S 1

Fig. 2

■ : P S K、　● : S 1

Fig. 3

:PSK、 ●:S1

Fig. 4

:PSK、 ●:S1

Fig. 5

BINDING ACTIVITY TO ACTIVE TGF-$\beta_1$ (%)

ACTIVE SUBSTANCES ($\mu$g/ml)

■ : P S K 、　● : S 1

Fig. 6

BINDING ACTIVITY TO PDGF (%)

ACTIVE SUBSTANCES ($\mu$g/ml)

■ : P S K 、　● : S 1

**European Patent Office**

**EUROPEAN SEARCH REPORT**

Application Number

EP 96 10 1623

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| E | EP-A-0 702 028 (KUREHA) 20 March 1996<br><br>* the whole document *<br>--- | 1-5,7-9,<br>12,13 | C07K14/375<br>A61K38/16<br>C12P21/00 |
| X | EP-A-0 353 861 (KUREHA) 7 February 1990<br><br>* the whole document *<br>--- | 1-5,7-9,<br>12,13 | |
| X | EP-A-0 058 093 (KUREHA) 18 August 1982<br>* the whole document *<br>--- | 1-5,7-13 | |
| X | WO-A-94 12199 (US DEPARTMENT OF THE ARMY )<br>9 June 1994<br>* the whole document *<br>--- | 1-5,7-9 | |
| X | DE-A-34 29 551 (KUREHA) 21 February 1985<br>* the whole document *<br>--- | 1-5,7-9 | |
| X | CHEMICAL ABSTRACTS, vol. 116, no. 15,<br>13 April 1992<br>Columbus, Ohio, US;<br>abstract no. 143415p,<br>Y KURAKATA ET AL.: "Functional maturation<br>of monocytes/macrphages induced by PSK<br>subfractions"<br>page 29;<br>XP002003222<br>* abstract *<br>& ANTICANCER RES. ,<br>vol. 11, no. 5, 1991,<br>pages 1767-1772,<br>----- | 1-5,7-9,<br>11,13 | TECHNICAL FIELDS SEARCHED (Int.Cl.6)<br><br>C07K<br>A61K<br>C12P |

The present search report has been drawn up for all claims

| Place of search | Date of completion of the search | Examiner |
|---|---|---|
| THE HAGUE | 21 May 1996 | Masturzo, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

..............................................................................

& : member of the same patent family, corresponding document

EPO FORM 1503 03.82 (P04C01)